# EUROPÄISCHE PATENTSCHRIFT

(11) **EP 0 362 824 B1**
(45) Veröffentlichungstag und Bekanntmachung des Hinweises auf die Patenterteilung: **19.01.1994**
(21) Anmeldenummer: 89118415.2
(22) Anmeldetag: 04.10.1989
(51) Int. Cl.: A61B 5/03

(54) **Druckmesskatheter**
Catheter for pressure measurement
Cathéter de la mesure de pression

(30) Priorität: 04.10.1988 DE 3833723
(43) Veröffentlichungstag der Anmeldung: 11.04.1990
(73) Patentinhaber: pvb medizintechnik gmbh, 85610 Kirchseeon (DE)
(72) Erfinder: Von Berg, Peter, D-8011 Neukeferloh (DE)
(74) Vertreter: von Bülow, Tam, Dr.

(56) Entgegenhaltungen:
- EP-A- 0 097 454
- DE-A- 3 130 367
- US-A- 3 703 099
- US-A- 4 274 423

## Beschreibung

Die Erfindung bezieht sich auf einen Druckmeßkatheter gemäß dem Oberbegriff des Patentanspruches 1. Ein solcher Druckmeßkatheter ist aus der US-A-3,703,099 bekannt. Dieser Katheter hat eine rohrförmige Meßspitze, deren freies Ende mit zwei gegenüberliegenden, axial verlaufenden Schlitzen versehen ist. An dem dem freien Ende der Meßspitze gegenüberliegenden Ende der Schlitze ist ein Druckmeßfühler angeordnet. Die Meßspitze ist von einem aufblasbaren Ballon umgeben. Ist der Ballon nicht aufgeblasen, so liegt er eng an der Meßspitze an. In Abhängigkeit vom herrschenden Druck an der Außenseite der Meßspitze werden die durch die Schlitze gebildeten Schenkel elastisch verformt, wobei diese Verformung auf den Druckmeßfühler übertragen und in ein elektrisches Meßsignal umgewandelt wird. Für einen "Nullabgleich" wird der Ballon mit einem unter Druck stehenden Gas aufgeblasen, so daß die beiden Schenkel am freien Ende der Meßspitze vom Umgebungsdruck abgekoppelt sind und sich in eine neutrale Stellung bewegen.

Die DE-A-31 30 367 zeigt einen Druckmeßkatheter, der am proximalen Ende offen ist und in der Nähe dieses Endes eine Druckmeßzelle aufweist. Das freie Ende des Katheters kann durch einen aufblasbaren Ballon verschlossen werden und wird über einen separaten, im Inneren des Katheters verlaufenden Kanal mit Druckluft beaufschlagt. In diesem Zustand ist die Meßzelle vom Außendruck abgekoppelt und kann mit einem bekannten Referenzdruck aus einer Infusionslösungsflasche beaufschlagt werden.

In der Geburtshilfe, aber auch in der Forschung, wird zur genauen Überwachung der Wehentätigkeit ein Druckmeßkatheter durch die Zervix in den Uterus eingeführt, um die dort auftretenden Druckschwankungen nach Änderung und (gegenüber dem außerhalb des Uterus vorliegenden Umgebungsdruck) relativer Größe zu erfassen und in Signale umzuwandeln, die einem Bildschirmgerät zur Anzeige und gegebenenfalls einem Aufzeichnungsgerät zur Registrierung zugeführt werden.

Hierbei ist wie bei jedem Einsatz eines Meßfühlers grundsätzlich bei der Inbetriebnahme des Druckmeßkatheters eine Eichung erforderlich, um systeminhärente, innere und gegebenenfalls auch durch Randbedingungen verursachte, äußere Meßwertverfälschungen mindestens an einem Eichpunkt zu kompensieren.

Grundsätzlich könnte diese Eichung bei jedem geeigneten Druck durchgeführt werden, doch man zieht in der Regel den Umgebungsdruck (atmosphärischen Druck) vor, bei welchem der zu messende relative Druck Null beträgt. Daher spricht man in diesem Fall nicht von einer Eichung schlechthin, sondern vielmehr von einem Nullpunktabgleich. Hierbei wird der Umgebungsdruck als Bezugs- oder Referenzdruck verwendet, und die angezeigte oder registrierte Differenz zum vom Druckmeßkatheter gemessenen Meßdruck wird durch Abgleichen der Anzeige- oder Registrierungseinrichtung auf Null eingestellt.

Es gibt zweierlei Arten von Druckmeßkathetern:
Bei der ersten wird der im Uterus vorliegende Druck durch eine Flüssigkeitssäule, die in einem Schlauch geführt ist, von einer durch eine Membran isolierten proximalen Meßstelle zu einem distalen Druckmeßfühler übertragen, der seinerseits den Signalgeber der Meßanordnung bildet.

Der Druckmeßfühler kann auch dann, wenn sich der Druckmeßkatheter in situ befindet, bei entsprechender Ausbildung ohne weiteres vom Katheter abgenommen werden, so daß ein Nullpunktabgleich des Meßfühlers stets vorgenommen werden kann. Neben einer Reihe hier weniger relevanter Nachteile hat ein solcher Druckmeßkatheter jedoch die Hauptnachteile, daß Blasen in der Druckübertragungsflüssigkeit dämpfend und somit verfälschend wirken, und daß der hydrostatische Meßfehler, der aus der Höhendifferenz zwischen der proximalen Meßspitze und dem Druckmeßfühler resultiert, nur ungenau abgeschätzt werden kann, weil die genaue Lage der genannten Meßspitze nicht bekannt ist oder sich wegen Körperbewegungen der Patientin verlagert.

Zum Vermeiden dieser Nachteile wurde daher ein Druckmeßkatheter einer zweiten Art vorgeschlagen, bei welchem sich der Druckmeßfühler nicht am distalen, sondern am proximalen Ende des Katheterschlauches und somit genau an der Meßstelle befindet. Ein solcher Druckmeßkatheter wird beispielsweise von der Firma Utah Medical Products. Inc. unter dem Namen "INTRAN" vertrieben.

Wie aus der von der genannten Firma zu diesem Druckmeßkatheter veröffentlichen Betriebsanleitung hervorgeht, wird der Nullpunktabgleich vor dem Einführen des Katheters vorgenommen, solange dessen Meßspitze dem atmosphärischen Umgebungsdruck ausgesetzt ist. Ferner wird in dieser Betriebsanleitung ausgeführt, daß ein neuer Nullpunktabgleich dann, wenn sich die Meßspitze in situ befindet, nicht erforderlich ist.

Dies ist jedoch nur solange richtig, als keine Störungen auftreten; fällt jedoch während einer Messung, die sich über Stunden erstrecken kann, das Anzeige- oder Registrierungsgerät durch Geräteschaden oder Bedienungsfehler (z.B. versehentliches Lösen eines Netzanschlußkabels beim Anschluß eines anderen Gerätes) aus, dann ist ein neuer Nullabgleich unabdingbar. Zu diesem müßte aber die Meßspitze des Katheters wieder an die Umgebungsluft gebracht werden, um dort dem atmosphärischen Druck ausgesetzt zu sein. Da die Meßspitze nach einer solchen Prozedur natürlich nicht mehr steril wäre, wird gleich ein neuer, steriler Druckmeßkatheter dem Nullpunktabgleich unter sterilen Bedingungen unterzogen und anstelle des bisherigen Katheters, der entfernt werden muß, in die Zervix eingeführt. Daß ein solches Verfahren umständlich und aufwendig ist, erübrigt der näheren Erörterung, zumal dieses Verfahren unter Umständen dann erforderlich ist, wenn der Verlauf der zu überwachenden Geburt einen rapiden und oft dramatischen Fortgang nimmt, so daß das Hantieren mit einem Katheter höchst störend sein kann.

Ein weiterer Nachteil des bekannten Katheters liegt darin, daß die sterile, den Katheter umschließende Verpackung zum Nullpunktabgleich geöffnet werden muß, so daß der Katheter während des Nullpunktabgleichs mindestens teilweise offenliegt und somit der Kontamination ausgesetzt ist, so daß ein zusätzliches Sterilitätsproblem auftritt.

Ein Druckmeßkatheter der genannten Art findet nicht nur in der Humanmedizin und in der Geburtshilfe Anwendung, sondern überall dort, wo das Auftreten von erheblichen Drucken in Körperhöhlungen von Mensch und Tier überwacht werden soll, die für einen Druckmeßfühler zugänglich sind, etwa im Pansen eines Wiederkäuers oder in Blutbahnen.

Angesichts dieser Problemlage liegt der Erfindung die Aufgabe zugrunde, den Druckmeßkatheter der eingangs genannten Art dahingehend zu verbessern, daß er mit einem Referenzdruck beaufschlagt werden kann.

Diese Aufgabe wird durch die im Anspruch 1 angegebenenen Merkmale gelöst. Vorteilhafte Ausgestaltungen und Weiterbildungen sind in den abhängigen Ansprüchen angegeben.

Es wird ein definierter Referenzdruck erzeugt und dem Druckmeßfühler zugeführt, etwa durch einen Kanal im Katheterschlauch; dieser Referenzdruck kann dem Druckmeßfühler anstelle des innerhalb des Uterus herrschenden Drucks zugeführt werden und kann als absoluter Druck oder in Relation zu einem während der Messung ständig verwendeten Bezugsdruck als Grundlage des Abgleiches verwendet werden.

Der in Anspruch 1 beanspruchte Druckmeßkatheter benutzt dieses Prinzip und weist einen Druckmeßfühler auf, der mit einem Meßdruckanschluß versehen ist, welcher in druckübertragender Weise mit der Außenseite der Meßspitze in Verbindung steht, sowie einem Bezugsanschluß, der über eine offene Höhlung im Katheterschlauch mit dessen distalem Ende und somit mit einer Bezugdruckquelle in Verbindung steht, die üblicherweise bei einem solchen Katheter von der Umgebungsluft gebildet wird.

Es ist eine Einrichtung zum Erzeugen eines Referenzdruckes an den Meßdruckanschluß anschließbar, während dieser von seiner druckübertragenden Verbindung mit der Außenseite der Meßspitze abgekoppelt ist; es herrscht somit in diesem Zustand zwischen dem Meßdruckanschluß des Druckmeßfühlers und dessen Bezugsanschluß kein Druckgefälle, da die erwähnten Drücke einander gleich sind.

Die Einrichtung zum Erzeugen des Referenzdruckes mündet in einen Strömungsmittelanschluß, der zwischen dem Meßdruckanschluß und der diesen nach außen abdeckenden Membrane angeordnet ist, so daß dann, wenn der Referenzdruck den außerhalb der Meßspitze und somit der Membrane vorliegenden Druck übersteigt, die Membrane von dem Druckmeßanschluß abgespreizt wird und die Druckübertragung von der Außenseite der Meßspitze auf den Druckmeßfühler unterbleibt.

Zum Erzeugen des Referenzdruckes kann eine Flüssigkeit verwendet werden; gemäß der Ausgestaltung der Erfindung nach Anspruch 4 ist die Einrichtung zum Erzeugen des Referenzdruckes jedoch als Drucklufterzeuger ausgebildet, da die zwischen die Membrane und den Druckmeßfühler eingeblasene Druckluft dort nach dem erfolgten Nullpunktabgleich keine Rückstände hinterläßt, die den weiteren Meßbetrieb beeinträchtigen könnten. Dies ist besonders dann von Vorteil, wenn der Meßfühler und die Membrane nicht unmittelbar miteinander in Verbindung stehen, sondern nur mittelbar und über beispielsweise ein Silikongelpolster, das den Druckmeßfühler vor grober mechanischer Belastung schützt, wenn der Katheter eingeführt wird. Hierbei liegt ein besonderer Vorteil der erfindungsgemäßen Ausgestaltung darin, daß bei dem Zusamenbau des Katheters oder bei dessen Einführen die Membrane gegenüber dem genannten Schutzpolster seitlich verschoben werden kann, so daß sie dann ständig den Meßwert verfälschende Scherkräfte auf das Schutzpolster und somit den Druckmeßfühler aufbringt. Durch die erfindungsgemäße Ausgestaltung sorgt das Abspreizen der Membrane gegenüber dem Schutzpolster dafür, daß alle bei der Übertragung des Meßdruckes beteiligten Elemente vor Beginn der Messung ihre Ausgangslage einnehmen können. Gleichzeitig erfolgt der Nullabgleich bei bereits eingeführtem Katheter.

Da der Nullabgleich der Anzeige- oder Aufzeichnungseinrichtung nur sehr kurze Zeit in Anspruch nimmt, muß auch der Referenzdruck über eine nur sehr kurze Zeit aufrechterhalten werden, so daß auch die der Meßspitze zugeführte Luftmenge gering ist. Dies ist deshalb wichtig, weil bei undichter Meßspitze sonst kontaminierte Luft in den Uterus gelangen könnte. Um dies zu vermeiden, ist gemäß Anspruch 4 vorgeschlagen, daß der Drucklufterzeuger als Kolben-Injektionspritze ausgebildet ist; diese kann in bereits aufgezogenem Zustand sterilisiert werden oder in diesem Zustand gemeinsam mit dem erfindungsgemäßen Katheter sterilisiert und verpackt sein, so daß beim Nullabgleich nur sterile Luft, in jedem Falle aber nur wenig Luft in die Meßspitze gelangt.

Bei jedem späteren erneuten Nullabgleich kann die Injektionsspritze durch eine neue, mit steriler Luft gefüllte ersetzt werden.

Der Bezugsanschluß des Druckmeßfühlers ist mit dem Strömungsmittelanschluß für den Referenzdruck verbunden, so daß der Druckmeßfühler dann, wenn die Membrane von ihm abgespreizt ist, an seinen beiden Anschlüssen mit dem gleichen Druck beaufschlagt wird.

Da der Differenzdruck zwischen den beiden Anschlüssen des Druckmeßfühlers Null wird, ist der Nullabgleich dann unmittelbar und ohne jeden weiteren Vergleich möglich.

Der Verbindungskanal, der den Bezugsanschluß mit der Einrichtung zum Erzeugen des Referenzdruckes verbindet, die ihrerseits in der Betriebsart zum Nullabgleich mit dem Meßdruckanschluß des Druckmeßfühlers verbunden ist, ist in der Meßspitze ausgebildet; während des Nullabgleichs muß nämlich der Druck an beiden Seiten des Druckmeßfühlers ausgeglichen sein; da dieser Nullabgleich sehr rasch erfolgt, könnte jedoch nach dessen Abschluß der Druckausgleich noch nicht abgeschlossen sein, wenn dieser über die offene Höhlung und eine zusätliche Druckleitung im Katheterschlauch erfolgen würde. Der in der Meßspitze angeordnete Verbindungskanal sorgt jedoch für einen praktisch augenblicklichen Druckausgleich, was den Nullabgleich noch weiter vereinfacht und genauer macht.

Eine Druckleitung im Katheterschlauch ist durch die für den Bezugsdruck ohnehin erforderliche offene Höhlung im Katheterschlauch gebildet. Hierbei wird beim Nullabgleich durch die Kolben-Injektionsspritze die in der offenen Höhlung vorhandene, sterile Luftsäule lediglich komprimiert bzw. verschoben, es wird aber nicht der Meßspitze zusätzliche Luft zugeführt, die dann erst sterilisiert werden müßte. Somit ist es möglich, auch mit einer Kolben-Injektionsspritze, deren Inhalt nicht vorher sterilisiert wurde, mehrmals hintereinander den Innendruck in der Meßspitze zum Nullabgleich zu erhöhen, ohne daß deshalb kontaminierte Luft durch die Meßspitze gepumpt wird und infolge einer dort etwa vorliegenden Leckstelle vielleicht zu einer Infektion führen könnte.

Die Membrane ist als ein die Meßspitze umhüllender Ballon ausgebildet, der in seiner Ruhelage gegen die Meßspitze und dabei unmittelbar oder auch über ein Druckpolster mittelbar gegen den Druckmeßfühler anliegt. Dieser kleine Ballon ist vom distalen Ende des Katheters her z.B. mittels einer Kolben-Injektionsspritze aufblasbar, wobei er sich dann vom Druckmeßfühler abhebt. Der besondere Vorteil der Ausbildung der Membrane als Ballon liegt darin, daß auch bei heftiger und zu starker Druckbeaufschlagung der Meßspitze durch den Referenzdruck, die bewirken könnte, eine in ein Fenster eingesetzte Membrane zu beschädigen oder herauszublasen, stets die Dichtigkeit der Meßspitze gewährleistet bleibt, da der Ballon sich zwar bereits bei Beaufschlagung durch eine geringe Luftmenge in ausreichender Weise vom Druckmeßfühler abwölbt, dagegen aber imstande ist, auch eine ganz beträchtlich größere Luftmenge aufzunehmen. Eine in die vom Ballon umgebene Außenoberfläche der Meßspitze einmündende, in deren Innerem mit der offenen Höhlung im Katheterschlauch in Verbindung stehende Öffnung leitet dem Inneren des Ballons den Referenzdruck zu.

Diese Öffnung ist als länglicher, an den Meßdruckanschluß des Druckmeßfühlers anschließender Schlitz ausgebildet, wodurch erreicht wird, daß der dem Meßdruckanschluß gegenüberliegende Teil des Ballons als erster mit Luft beaufschlagt und somit aufgebläht und vom Druckmeßfühler abgewölbt wird.

Der Druckmeßfühler ist bevorzugt als Dehnungsmeßelement ausgebildet; der Ballon besteht bevorzugt aus einem Elastomer mit hohem Rückstellvermögen; die übrigen Teile des erfindungsgemäßen Druckmeßkatheters bestehen, soweit sie mit dem zu untersuchenden Körper in Berührung gelangen, aus physiologisch unbedenklichen Kunststoffen.

Bevorzugt weist der Katheterschlauch neben der offenen Höhlung, die als einfacher Kanal ausgebildet sein kann, auch einen Kanal zur Aufnahme der elektrischen Leitungen auf, sowie bevorzugt einen weiteren Kanal, der zur Außenseite des Katheterschlauches hin an einer Stelle nahe dem Ballon offen ist und dazu dient, daß Behandlungsflüssigkeit in den Uterus eingeleitet werden kann oder Flüssigkeitsproben entnommen werden können, während sich die Meßspitze in situ befindet.

Der Gegenstand der Erfindung wird anhand der beigefügten, schematischen Zeichnung beispielsweise noch näher erläutert.
In dieser zeigt:
Fig. 1 die Ansicht des distalen Endes einer Ausführungsform eines erfindungsgemäßen Druckmeßkatheters.
Fig. 2 die Meßspitze (das proximale Ende) der in Fig. 1 gezeigten Ausführungsform, im Längsschnitt und in vergrößertem Maßstab, und
Fig. 3 eine Draufsicht auf die in Fig. 2 gezeigte Meßspitze, mit angedeutetem Querschnitt des Katheterschlauches.

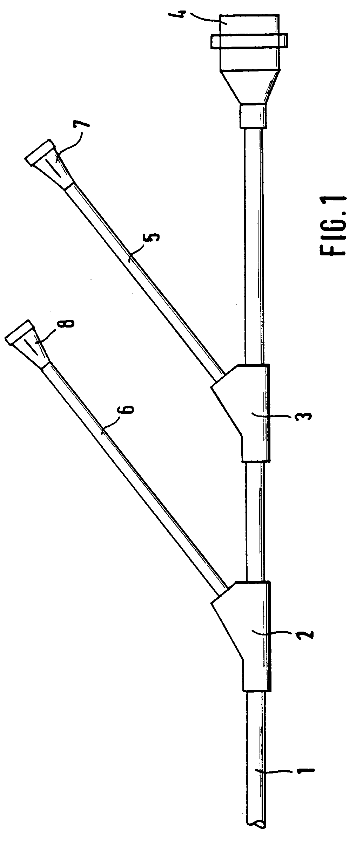

In Fig. 1 ist in etwa natürlichem Maßstab die Draufsicht auf das distale Ende eines erfindungsgemäßen Druckmeßkkatheters für die intrauterine Druckmessung gezeigt, mit einem Endabschnitt des Katheterschlauches 1, einem ersten Abzweigungsstück 2, an dem ein Infusionsschlauch 6 von dem mehrkanaligen Katheterschlauch 1 abzweigt, und mit einem zweiten Abzweigungsstück 3, an dem ein Luftschlauch 5 vom Katheterschlauch 1 abzweigt. Das Ende des Katheterschlauches 1 mündet in eine elektrische Kupplung 4 ein, die zum Anschluß an das Eingangskabel (nicht gezeigt) eines Monitors oder Oszillographen (nicht gezeigt) o. dergl. eingerichtet ist.

Am freien Ende des Infusionsschlauches 6 sitzt eine Spritzenkupplung 8 zum Anschluß an eine Injektionsspritze, eine mechanische Spritzen-Dosiereinrichtung oder eine Tropfinfusionseinrichtung (nicht gezeigt), während am freien Ende des Luftschlauches 5 eine Spritzenkupplung 7 zum Anschluß an eine luftgefüllte Injektionsspritze (nicht gezeigt) sitzt. In normalen Betrieb bleibt die Spritzenkupplung 7 offen.

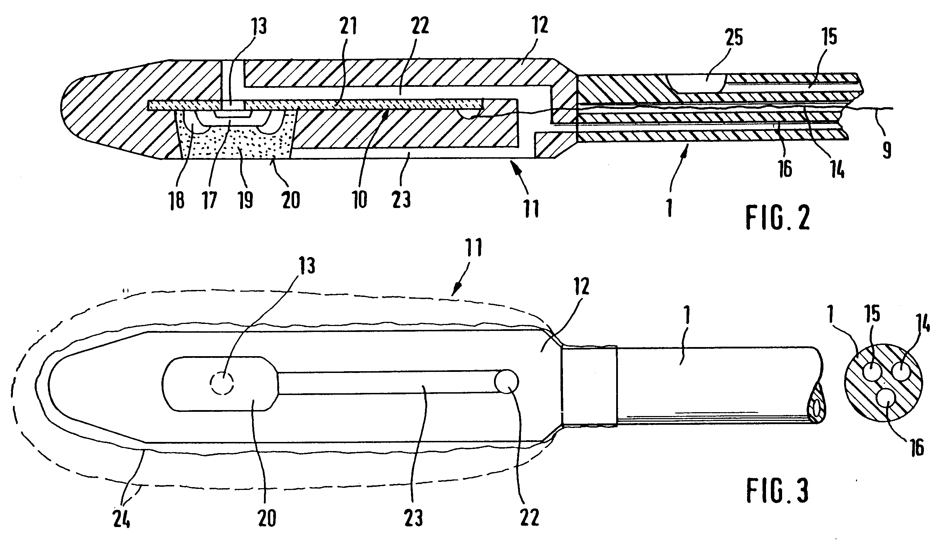

Der Luftschlauch 5 ist mit einem Luftkanal 16 im Katheterschlauch 1 verbunden (sh. Fig. 2 und 3), der Infusionsschlauch 6 mit einem Infusionskanal 15 und die elektrische Kupplung 4 sitzt am Ende eines Kabelkanales 14, in dem die elektrischen Anschlußkabel 9 verlegt sind, die von einem Druckmeßfühler 10 (Fig. 2) ausgehen.

Wie in Fig. 2 und 3 gezeigt, ist am proximalen Ende des Katheterschlauches 1 eine Meßspitze 11 angesetzt, die von einem Träger 12 aus Kunststoff gebildet ist, der die noch zu beschreibenden, übrigen Teile aufnimmt und ebenso wie die Schläuche 1, 5 und 6 aus Polyethylen oder Polypropylen gebildet ist.

Der Druckmeßfühler 10 weist einen länglichen Keramikträger 21 auf, der in den Träger 12 eingebettet ist und an dessen dem Katheterschlauch 1 zugewandtem Ende die Anschlüsse für elektrische Anschlußkabel 9 sitzen, während am anderen Ende des Keramikträgers 21 eine Durchgangsöffnung 13 ausgespart ist, die auf der einen, in Fig. 2 unteren Seite des Keramikträgers 21 von einem napfförmigen Dehnungsfühler 17 dichtend abgedeckt ist. Wenn nun zwischen der Durchgangsöffnung 13 und der Unterseite des Dehnungsfühlers 17 eine Druckdifferenz, beispielsweise ein Unterdruck, auftritt, dann wölbt sich der Dehnungsfühler, in diesem Fall nach oben und ändert hierbei seinen elektrischen Widerstand oder seine elektrische Kapazität bzw. gibt ein Signal ab.

Der Dehnungsfühler 17 sitzt in einer Aussparung 18 des Trägers 12, die mit einem Schutzpolster 19 aus Silikongel weitgehend ausgefüllt ist, das sich zur Druckübertragung auf den Dehnungsfühler 17 wie eine Flüssigkeit verhält, aber Punktbelastungen absorbiert, die den Dehnungsfühler 17 sofort schädigen könnten.

Die außenliegende Stirnfläche des Schutzpolsters 19, die bündig mit der Außenoberfläche des Trägers 12 abschließt, bildet den Meßdruckanschluß des Druckmeßfühlers 10, währed die Durchgangsöffnung 13 dessen Bezugsanschluß bildet.

Im Inneren des Trägers 12 ist ein Ausgleichsraum ausgespart, der die Durchgangsöffnung 13 mit der nächstliegenden Außenoberfläche des Trägers 12 sowie mit dem Luftkanal 16 im Katheterschlauch 1 verbindet. Ferner ist der Ausgleichsraum mit einem Längsschlitz 23 verbunden, der sich über nahezu die gesamte Länge des Trägers 12 erstreckt und nahe dem Meßdruckanschluß 20 endet.

Über den Träger 12 ist, wie aus Fig. 3 ersichtlich, ein diesen eng umschließender Ballon 24 aufgeschoben und am proximalen Ende des Katheterschlauches 1 dichtend befestigt.

In dem druckfreien Zustand nimmt der Ballon 24 jene Lage ein, die in Fig. 3 ausgezogen gezeigt ist, wobei er insbesondere als druckübertragende Membrane gegen den Meßddruckanschluß 20 anliegt.

In aufgeblasenem Zustand, der weiter unten noch näher erörtert wird, nimmt der Ballon die in Fig. 3 gestrichelt gezeigte Lage ein, wobei er insbesondere vom Meßdduckanschluß 20 abhebt.

Da der Ballon 24 aus mindestens ein wenig durchsichtigem Silikongummi besteht, sind der Meßdruckanschluß 20 und der Schlitz 23 in Fig. 3 sichtbar.

Fig. 2 zeigt noch eine Infusionsöffnung 25 die nahe dem proximalen Ende des Katheterschlauches 1 in dessen Wand ausgebildet ist und einen Auslaß für den Infusionskanal 15 bildet.

Der gezeigte Druckmeßkatheter ist für den einmaligen Gebrauch bestimmt und in eine bakteriendichte Umhüllung steril eingeschweißt. Hierbei können die Spritzenkupplungen 7 und 8 durch lösbare Stopfen verschlossen sein; an der Spritzenkupplung 7 für den Luftschlauch 5 kann auch eine aufgezogene, mit steriler Luft gefüllte Einmalspritze lösbar angekuppelt sein.

Der Katheter wird bei der Benutzung unmittelbar nach der Entnahme aus der sterilen Verpackung sofort durch die Zervix einer Patientin mit seiner Meßspitze 11 in deren Uterus eingeführt und gegebenenfalls unter Ultraschallkontrolle plaziert. Falls erforderlich, wird der Katheterschlauch 1 an der Patientin fixiert.

Nun wird der Katheter mit dem elektrischen Anschluß 4 an einen Monitor oder einen Plotter o. dergl. angeschlossen. Dann wird bei der an der Spritzenkupplung 7 von vorneherein oder inzwischen angekoppelten, luftgefüllten Kolbenspritze der Kolben langsam und vollständig niedergedrückt; gegen Ende dieser Tätigkeit wird der an dem genannten Beobachtungs- oder Aufzeichnungsgerät vorgesehene Knopf für den Nullabgleich gedrückt oder der Nullabgleich wird in der sonstwie vorgesehenen Weise durchgeführt. Dann wird die Kolbenspritze von der Kupplung 7 gelöst und entfernt.

Beim Niederdrücken des Kolbens der Kolbenspritze wird der Druck in der Luftleitung 16 stark erhöht und gelangt über den Ausgleichsraum 22 in der Meßspitze 11 und dessen Verbindungsöffnungen zur Außenseite des Trägers 12 und somit zur Innenseite des Ballons 24, der hierdurch aufgeblasen wird, wie gestrichelt in Fig. 3 gezeigt. Hierbei liegt der im Inneren des Ballons herrschende, gleichmäßige Luftdruck sowohl an dem Meßdruckanschluß 20, von dem sich der Ballon 24 gelöst hat, wie auch am Bezugsanschluß 13 an, so daß der Dehnungsfühler 17 einem Differenzdruck in der Größe Null ausgesetzt ist. Währenddessen wird die nachgeschaltete Anzeige- oder Aufzeichnungseinrichtung abgeglichen; die Anzeige muß nun den Relativdruck Null zeigen.

Nach dem Entfernen der Spritze von der Kupplung 7 fällt der Druck im Luftkanal 16 und damit auch im Inneren des Ballons 24 ab, bis der Luftdruck am Bezugsanschluß 13, im Luftkanal 16 und im Luftschlauch 5 dem Atmosphärendruck entspricht und der Ballon 24 eng gegen die Außenoberfläche des Trägers 12 anliegt.

Ist ein nachträglicher Druckabgleich erforderlich, weil etwa der bisher nur auf einem Bildschirm angezeigte Druckverlauf durch ein zusätzliches Gerät auch aufgezeichnet werden soll, dann wird eine luftgefüllte, aufgezogene Spritze an die Kupplung 7 angeschlossen, und der oben beschriebene Nullabgleich wird nochmals durchgeführt.

Somit ist es möglich, den Nullabgleich so oft wie erforderlich durchzuführen, während die Meßspitze 11 des Katheters von Anfang an in situ verbleibt. Die Infektionsgefahr ist somit auf ein Mindestmaß verringert, während gleichzeitig sehr genaue Messungen erzielt werden können, da die einzige variable Störgröße, die bei der Messung auftritt, der statische Flüssigkeitsdruck im Inneren des Uterus ist.

## Patentansprüche

1. Druckmeßkatheter mit einem an seinen beiden Enden offenen hohlen Katheterschlauch (1) und einer an dessen proximalen Ende angebrachten Meßspitze (11), in deren Inneren eine Druckmeßfühleranordnung (10) angeordnet ist, die einen Meßdruckanschluß und einen Bezugsdruckanschluß (13) sowie ein sich durch den Katheterschlauch (1) bis zu dessen distalen Ende hin erstreckendes elektrisches Anschlußkabel (9) aufweist, wobei der Meßdruckanschluß (20) über eine Membrane mit der Außenoberfläche der Meßspitze und der Bezugsdruckanschluß (13) mit dem Inneren des Katheterschlauches (13) in Verbindung steht, wobei die Meßspitze von einem aufblasbaren dünnwandigen Ballon (24) umgeben ist, der in nicht aufgeblasenem Zustand die Membran bildet und in aufgeblasenem Zustand den Meßdruckanschluß (20) von der Druck übertragenden Verbindung mit der Außenoberfläche der Meßspitze (11) abkoppelt und an eine Einrichtung zum Erzeugen eines Referenzdruckes anschließt und wobei im Inneren der Meßspitze (11) eine den Bezugsanschluß (13) des Druckmeßfühlers (10) mit einer offenen Höhlung (16) im Katheterschlauch (1) verbindende Aussparung (22) ausgebildet ist,
dadurch gekennzeichnet,
daß die Aussparung (22) durch mindestens eine einen Strömungsmittelanschluß bildende Öffnung mit der vom Ballon (24) umgebenen Außenoberfläche der Meßspitze (11) in Verbindung steht, wodurch dem Ballon (24) der Referenzdruck zugeleitet wird.

2. Druckmeßkatheter nach Anspruch 1, dadurch gekennzeichnet, daß die Aussparung (22) über den als Durchgangsöffnung (13) ausgebildeten Bezugsanschluß in der Meßspitze (11) mit dem Inneren des Ballons (24) verbunden ist.

3. Druckmeßkatheter nach Anspruch 1 oder 2, dadurch gekennzeichnet, daß auf der zur Meßseite weisenden Oberfläche der Meßspitze (11) ein Längsschlitz (23) verläuft, der mit einer im Inneren des Katheterschlauches (1) verlaufenden Druckleitung (16) verbunden ist.

4. Druckmeßkatheter nach einem der Ansprüche 1 bis 3, dadurch gekennzeichnet, daß an dem der Meßspitze (11) abgewandten Ende der Druckleitung (16) eine Kolben-Injektionsspritze als Drucklufterzeuger zum Erzeugen des Referenzdruckes angeschlossen ist.

## Claims

1. A pressure measuring catheter having a hollow catheter hose (1) open at both ends and a measuring tip (11) which is attached to the proximal end of the hose and in the interior of which there is arranged a pressure measuring sensor arrangement (10) which has a measuring pressure connection and a reference pressure connection (13) and an electric connecting cable (9) which extends through the catheter hose (1) as far as the distal end of the latter, the measuring pressure connection (20) being connected to the outer surface of the measuring tip via a membrane and the reference pressure connection (13) being connected to the interior of the catheter hose (13), the measuring tip being surrounded by a inflatable thin-walled balloon (24) which in the non-inflated state forms the membrane and in the inflated state disconnects the measuring pressure connection (20) from the pressure transmitting connection with the outer surface of the measuring tip (11) and connects it to a device for producing a reference pressure, and a hollow (22) connecting the reference connection (13) of the pressure measuring sensor (10) to an open cavity (16) in the catheter hose (1) being formed in the interior of the measuring tip (11), characterised in that the hollow (22) is connected to the measuring tip outer surface surrounded by the balloon (24) by means of at least one opening forming a flow means connection, whereby the reference pressure is supplied to the balloon (24).

2. A pressure measuring catheter in accordance with Claim 1, characterised in that the hollow (22) is connected to the interior of the balloon (24) via the reference connection located in the measuring tip (11) and formed as a through opening (13).

3. A pressure measuring catheter in accordance with Claim 1 or 2, characterised in that a longitudinal groove (23) runs in the measuring tip surface directed towards the measuring side, this longitudinal groove (23) being connected to a pressure line (16) running in the interior of the catheter hose (1).

4. A pressure measuring catheter in accordance with any one of Claims 1 to 3, characterised in that a piston syringe - as a compressed air generator for producing the reference pressure - is connected to the end of the pressure line (16) directed away from the measuring tip (11).

## Revendications

1. Catheter de mesure de pression muni d'un tube souple de catheter (1), creux et ouvert à ses deux extrémités et une pointe de mesure (11) montée à l'une de ses extrémités proximales, et à l'intérieur de laquelle est disposé un agencement de capteurs de mesure de pression (10), qui comporte un raccord de pression de mesure et un raccord de pression de référence (13), ainsi qu'un câble de connexion électrique (9) s'étendant à l'intérieur du tube souple de catheter (1) jusqu'à son extrémité distale, le raccord de pression de mesure (20) se trouvant en liaison par une membrane avec la surface extérieure de la pointe de mesure et le raccord de pression de référence (13) étant relié à l'intérieur du tube souple de catheter (13), la pointe de mesure étant entourée d'un ballon gonflable à paroi mince (24), qui à l'état non gonflé forme la membrane, et, à l'état gonflé, désaccouple le raccord de pression de mesure (20) appartenant à la liaison transmettant la pression de la surface extérieure de la pointe de mesure (11) et se raccorde à un dispositif de création d'une pression de référence et dans lequel est réalisé à l'intérieur de la pointe de mesure (11) un évidement reliant le raccord de référence (13) du capteur de mesure de pression (10) avec une cavité ouverte (16) dans le tube souple de catheter (1), caractérisé en ce que l'évidement (22) est relié par au moins une ouverture formant un raccord de moyens d'écoulement, à la surface extérieure de la pointe de mesure (1) qu'entoure le ballon (24), de sorte que la pression de référence soit amenée au ballon (24).

2. Catheter de mesure de pression selon la revendication 1, caractérisé en ce que l'évidement (22) est relié par le raccord de référence réalisé sous la forme d'une ouverture de passage (13), à l'intérieur de la pointe de mesure (11) avec l'intérieur du ballon (24).

3. Catheter de mesure de pression selon la revendication 1 ou 2, caractérisé en ce que sur la surface de la pointe de mesure (11) tournée vers la face de mesure, se développe une fente longitudinale (23) qui est reliée avec une conduite de pression (16) s'étendant à l'intérieur du tube souple de catheter (1).

4. Catheter de mesure de pression selon l'une des revendications 1 à 3, caractérisé en ce qu'à l'extrémité de la conduite de pression (16) opposée à la pointe de mesure 11, est raccordée une seringue d'injection à piston servant de générateur d'air comprimé pour créer la pression de référence.
